# EUROPEAN PATENT APPLICATION

(11) **EP 4 285 965 A1**
(43) Date of publication of application: **06.12.2023**
(21) Application number: 22305807.4
(22) Date of filing: 02.06.2022
(51) Int. Cl.: A61M 5/315, A61M 5/32, A61M 5/20

(54) **RIGID NEEDLE SHIELD REMOVER WITH CONNECTION CONFIRMATION FEATURE FOR AUTOINJECTOR**

(71) Applicant: Becton, Dickinson and Company, Franklin Lakes, NJ 07417-1880 (US)
(72) Inventor: DUCAROUGE, Pierre, 38100 Grenoble (FR); VALENTIN, Stéphane, 38470 L ALBENC (FR); LE DIMET, Gwenn, 38850 Charavines (FR); LAGEAT, Claire, 38100 Grenoble (FR)
(74) Representative: Germain Maureau

(57) **Abstract**

Provided herein is a subassembly for a drug delivery device, including a housing, a displaceable needle cover having a distal end having a flange, the needle cover being received within the housing and configured to have a pre-use position in which the needle cover extends from the housing and a use position in which the needle cover is at least partially displaced into the housing. Displacement of the needle cover into the housing actuates the drug delivery device. The subassembly further includes a cap assembly including a push back element operatively received within the retainer, wherein the push back element is configured to push the retainer in a proximal direction.

## Description

### BACKGROUND OF THE INVENTION

### Field of the Invention

The present disclosure relates generally to autoinjectors and methods of assembling the same and, more specifically, to devices for removing needle shields from autoinjectors, devices for preventing unintended actuation of autoinjectors, and methods for assembling autoinjectors including such devices.

### Description of Related Art

Automatic drug delivery devices, such as autoinjectors, are designed with removable shields to maintain sterility of the needle and to prevent inadvertent contact between the needle and a user. Many of these needle shields are elastomeric, and ensuring accurate placement and a firm grip between the needle shield and caps or other removal devices can be difficult, resulting in a need for excess force to remove the shield or slippage and an inability to remove the shield at all. Accordingly, a need exists in the art for a needle shield remover that can reliably be positioned for quick and easy removal of the needle shield when the autoinjector is to be used.

In addition, the removable shields of these autoinjectors often include a cavity on a distal end thereof to permit users to push the cap of the removable shields in a proximal direction to ensure proper connection between the rigid needle shield and retainer legs used to remove the rigid needle shield. In order to ensure proper removal of the rigid needle shield from the autoinjector, the retainer legs should be in operative contact with the proximal end of the rigid needle shield to pull the rigid needle shield off of the autoinjection when the removable shield is pulled in a distal direction away from the autoinjector. To ensure the removable shield, and thereby the retainer legs, have been sufficiently pushed in the proximal direction to ensure proper positioning of the retainer legs relative to the rigid needle shield, a user will often push the distal end of the removable shield in the proximal direction. However, due to the cavity defined in the distal end of the removable shield, it is not always apparent to the user the proper location to press on or that the removable shield needs to be pressed at all to ensure proper positioning of the retainer legs. Currently, a push back pin may be used to push the retainer legs into position relative to the rigid needle shield. This push back pin is provided separately from the removable shield and is inserted into the cavity defined by the removable shield. Often, however, this removable push back pin may be lost or unavailable, leaving the user without the ability to push the retainer legs into position. Therefore, there is also a current need in the art for an improved method and arrangement for ensuring the retainer legs of the removable shield are properly positioned relative to a rigid needle shield on an autoinjector.

### SUMMARY OF THE INVENTION

Clause 1: In accordance with an aspect of the present invention, a subassembly for a drug delivery device includes a housing, a displaceable needle cover including a distal end including a flange, the needle cover received within the housing and configured to have a pre-use position in which the needle cover extends from the housing, and a use position in which the needle cover is at least partially displaced into the housing, wherein displacement of the needle cover into the housing actuates the drug delivery device through interaction with a drive mechanism. The subassembly also includes a cap assembly including a cap remover configured to be grasped by a user, a retainer received within the cap remover and configured to engage the flange of the needle cover of the drug delivery device, and a push back element operatively received within the retainer, wherein the push back element is configured to push the retainer in a proximal direction.
Clause 2: In accordance with an aspect of the present invention, the push back element comprises a tab and an extension member that extends from the tab.
Clause 3: In accordance with an aspect of the present invention, the push back element is removably held in the retainer.
Clause 4: In accordance with an aspect of the present invention, the push back element include the tab that covers an opening defined in the cap remover.
Clause 5: In accordance with an aspect of the present invention, the push back element includes the extension member configured to be received in the retainer, wherein at least a portion of the retainer defines an opening with a cross-sectional shape that corresponds to a cross-sectional shape of the extension member.
Clause 6: In accordance with an aspect of the present invention, the push back element is made of a medical-grade plastic.
Clause 7: In accordance with an aspect of the present invention, a proximal surface of the push back element is configured to push the retainer in the proximal direction.
Clause 8: In accordance with an aspect of the present invention, the push back element comprises the tab that is shaped to correspond to a shape of an opening defined in the cap remover.
Clause 9: In accordance with an aspect of the present invention, a drug delivery device includes a housing, a syringe including a needle and received at least partially within the housing, a needle cover including a flange at a distal end thereof and having a pre-use position in which the needle is positioned within the needle cover, and a use position in which the needle is positioned at least partially outside of the needle cover, wherein in the use position the needle cover is configured to actuate the drug delivery device to deliver a medicament. The drug delivery device also includes a cap assembly including a cap remover, a retainer received within the cap remover and a push back element operatively received within the retainer, wherein the push back element is configured to push the retainer in a proximal direction.
Clause 10: In accordance with an aspect of the present invention, the push back element includes a tab and an extension member that extends from the tab.
Clause 11: In accordance with an aspect of the present invention, the push back element is removably held in the retainer.
Clause 12: In accordance with an aspect of the present invention, the push back element includes the tab that covers an opening defined in the cap remover.
Clause 13: In accordance with an aspect of the present invention, the push back element includes the extension member configured to be received in the retainer, wherein at least a portion of the retainer defines an opening with a cross-sectional shape that corresponds to a cross-sectional shape of the extension member.
Clause 14: In accordance with an aspect of the present invention, the push back element is made of a medical-grade plastic.
Clause 15: In accordance with an aspect of the present invention, a cap assembly for a drug delivery device includes a cap remover configured to be grasped by a user, a retainer received within the cap remover and configured to engage the flange of the needle cover of the drug delivery device, and a push back element operatively received within the retainer, wherein the push back element is configured to push the retainer in a proximal direction.
Clause 16: In accordance with an aspect of the present invention, the push back element includes a tab and an extension member that extends from the tab.
Clause 17: In accordance with an aspect of the present invention, the push back element is removably held in the retainer.
Clause 18: In accordance with an aspect of the present invention, the push back element includes the tab that covers an opening defined in the cap remover.
Clause 19: In accordance with an aspect of the present invention, the push back element includes the extension member configured to be received in the retainer, wherein at least a portion of the retainer defines an opening with a cross-sectional shape that corresponds to a cross-sectional shape of the extension member.
Clause 20: In accordance with an aspect of the present invention, the push back element is made of a medical-grade plastic.

### BRIEF DESCRIPTION OF THE DRAWINGS

FIG. 1A is a side view of a cap assembly according to a non-limiting embodiment or aspect described herein;
FIG. 1B is a bottom view of a cap assembly according to a non-limiting embodiment or aspect described herein;
FIG. 2A is an exploded view of a cap assembly according to a non-limiting embodiment or aspect described herein;
FIG. 2B is a perspective view of the cap assembly as shown in FIG. 2A;
FIG. 3 is a cross-sectional view of a cap assembly according to a non-limiting embodiment or aspect described herein;
FIG. 4A is a perspective view of a cap assembly according to a non-limiting embodiment or aspect described herein;
FIG. 4B is a perspective view of a cap assembly according to a non-limiting embodiment or aspect described herein;
FIG. 5A is an exploded perspective view of a cap assembly according to a non-limiting embodiment or aspect described herein;
FIG. 5B is a perspective view of a cap remover of a cap assembly according to a non-limiting embodiment or aspect described herein;
FIG. 5C is a perspective view of a retainer of a cap assembly according to a non-limiting embodiment or aspect described herein;
FIG. 6 is a cross-sectional perspective view of a cap assembly according to a non-limiting embodiment or aspect described herein;
FIG. 7 is a cross-sectional view of an autoinjector including a cap assembly according to a non-limiting embodiment or aspect described herein;
FIG. 8 is a cross-sectional view of an autoinjector including a cap assembly according to a non-limiting embodiment or aspect described herein;
FIG. 9 is a cross-sectional view of an autoinjector including a cap assembly according to a non-limiting embodiment or aspect described herein;
FIG. 10 is a cross-section view of a subassembly including a cap assembly according to a non-limiting embodiment or aspect described herein;
FIGS. 11A-11J show steps of assembly of a subassembly according to a non-limiting embodiment or aspect described herein;
FIG. 12 shows a side view of an autoinjector according to a non-limiting embodiment or aspect described herein;
FIG. 13 is a cross-sectional view of an autoinjector according to a non-limiting embodiment or aspect described herein including a push back element; and
FIG. 14 is a perspective view of the push back element of FIG. 13.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

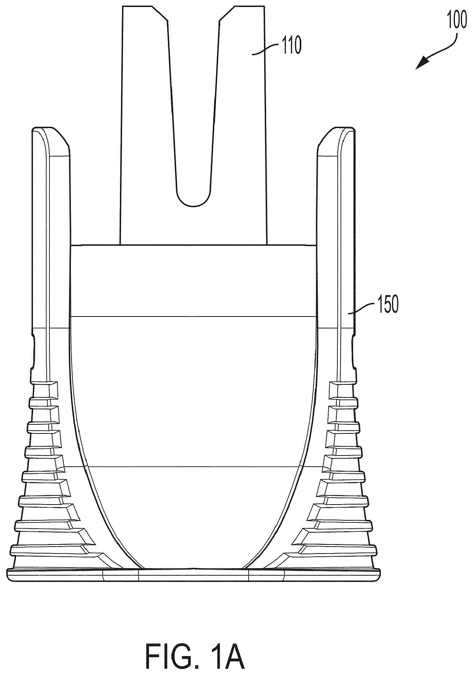

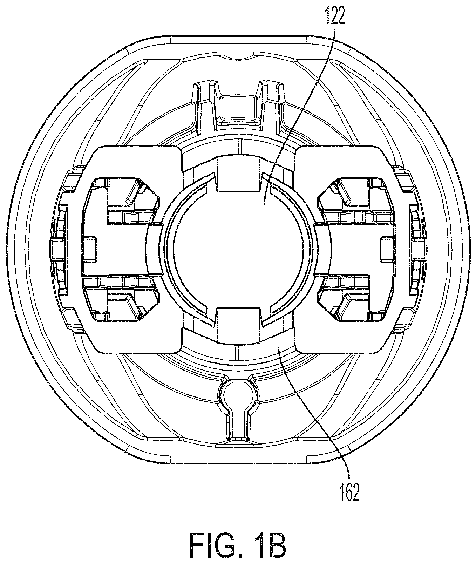

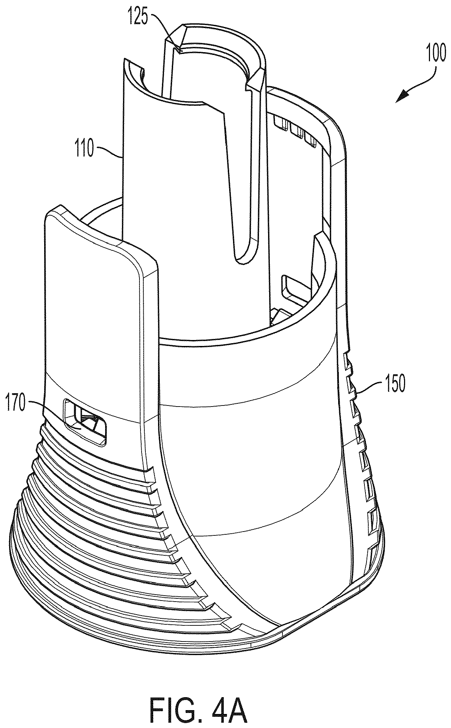

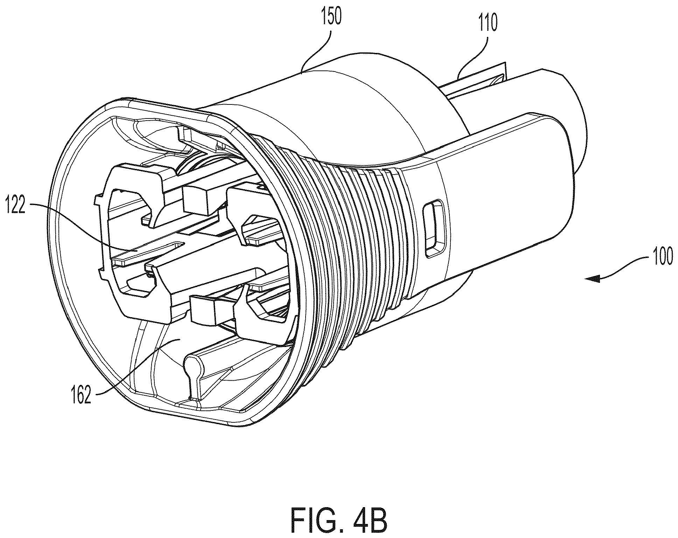

Provided herein is a cap assembly for a drug delivery device including a needle. In non-limiting embodiments or aspects, the drug delivery device is an autoinjector or other similar device for automatic or manual delivery of a substance to a user. With reference to **FIGS. 1-6****,** shown is a cap assembly (100), which in non-limiting embodiments or aspects is a shield removal assembly including a retainer (110) and a cap remover (150). Retainer (110) is nested within cap remover (150). In non-limiting embodiments or aspects, cap remover (150) is flared at a distal end thereof to form an ergonomic gripping surface to allow a user to firmly grip the cap remover. Retainer (110) and cap remover (150) can be formed of any rigid material, for example, and without limitation, plastics and the like.

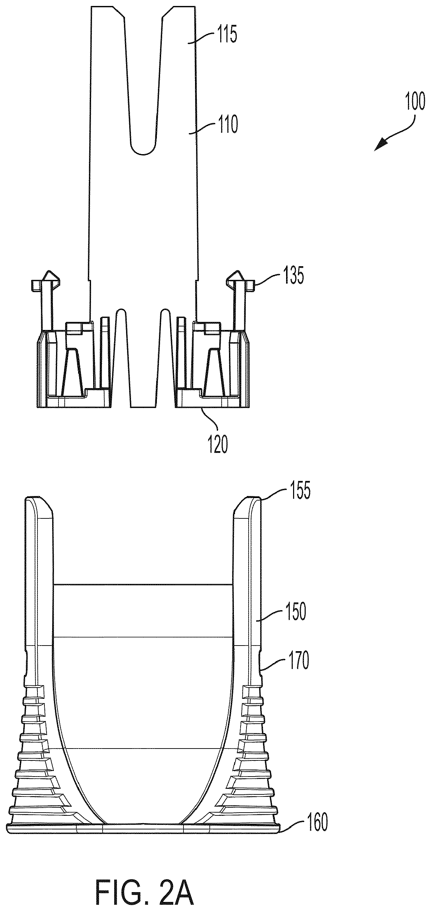

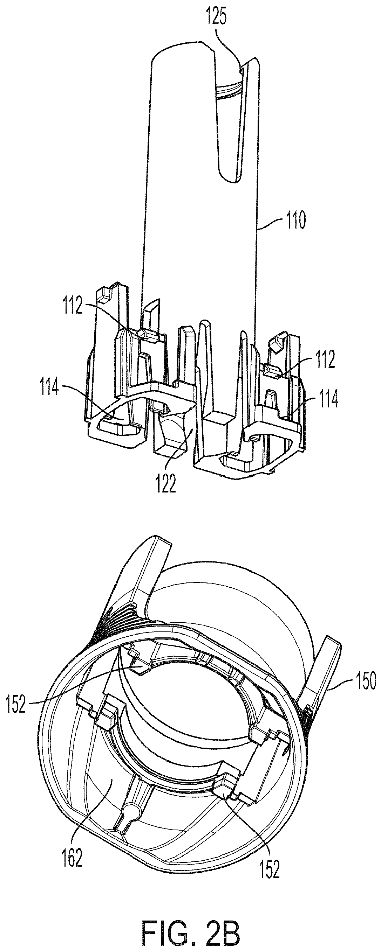

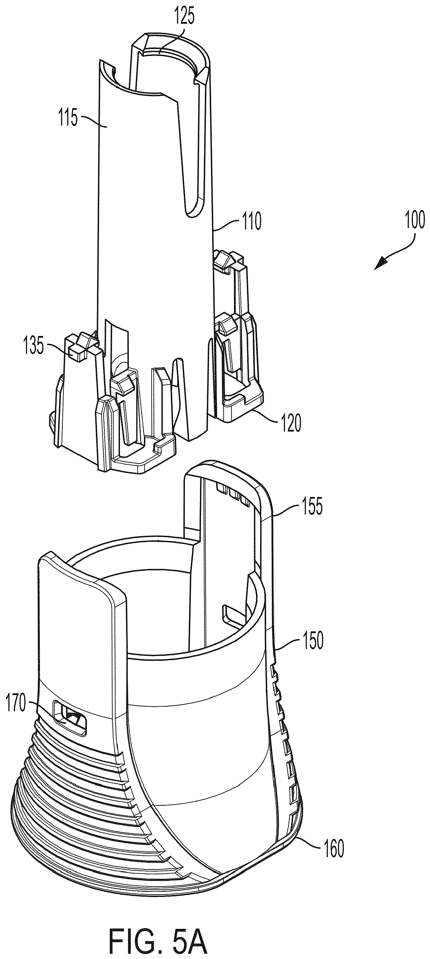

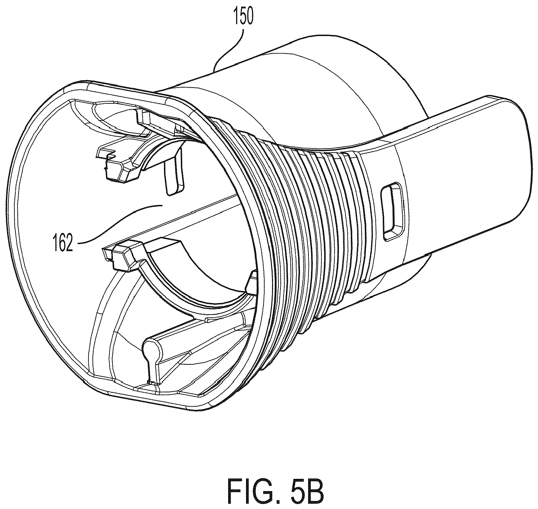

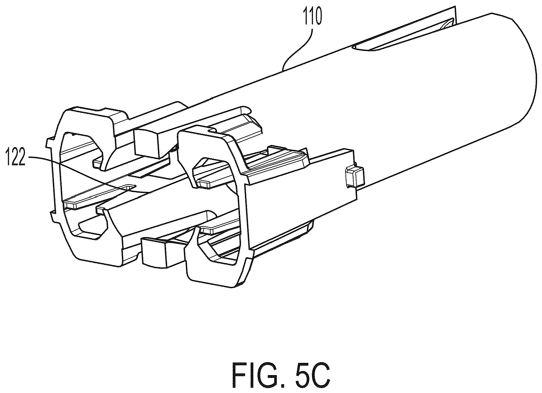

**FIGS. 2A****,** **2B****, and** **5A****-5C** show exploded views of a non-limiting embodiment or aspect of the cap assembly described herein. Cap remover (150) includes proximal end (155), which is configured to be closest to the housing, and a distal end (160). In non-limiting embodiments or aspects, proximal end (155) of cap remover (150) includes two or more arms that engage with a housing (not shown) of an autoinjector. In such non-limiting embodiments or aspects, the arms of the cap remover (150) include at least one projection or shoulder to allow secure, releasable engagement with the housing of the autoinjector. The distal end (160) of cap remover (150) includes at least one opening (162) therethrough. In non-limiting embodiments or aspects, cap remover (150) includes in a sidewall thereof at least one opening (170). In non-limiting embodiments or aspects, cap remover (150) includes two openings (170). In non-limiting embodiments or aspects, cap remover (150) includes two openings (170) that are located on opposite sides of the cap remover (150).

With continued reference to **FIGS. 1****,** **2A****,** **2B****,** **4****,** **5A****, and** **5B****,** retainer (110) includes proximal (115, closest to the housing) and distal (120) ends. In non-limiting embodiments or aspects, proximal end (115) of retainer (110) includes two or more arms that engage with the needle shield (not shown) of an autoinjector. In such non-limiting embodiments or aspects, the arms of the retainer (110) include at least one projection to allow secure engagement with the needle shield. The distal end (120) of retainer (110) includes at least one opening (122) therethrough. In non-limiting embodiments, the distal end (120) of the retainer (110) is configured to abut the distal end (160) of the cap remover (150). In non-limiting embodiments or aspects, the opening (162) in the distal end (160) of the cap remover (150) is of a larger diameter than the opening (122) in the distal end (120) of the retainer (110). In non-limiting embodiments or aspects, this difference in diameter allows the retainer (110) to be introduced into the cap remover (150) through the opening (162) in the distal end (160) of the cap remover (150).

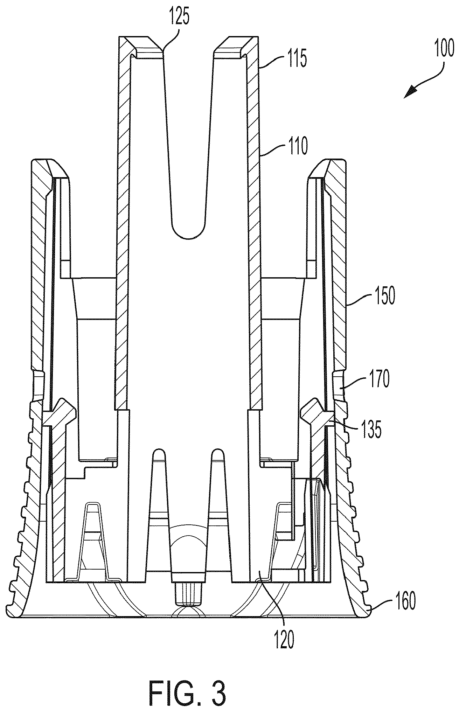

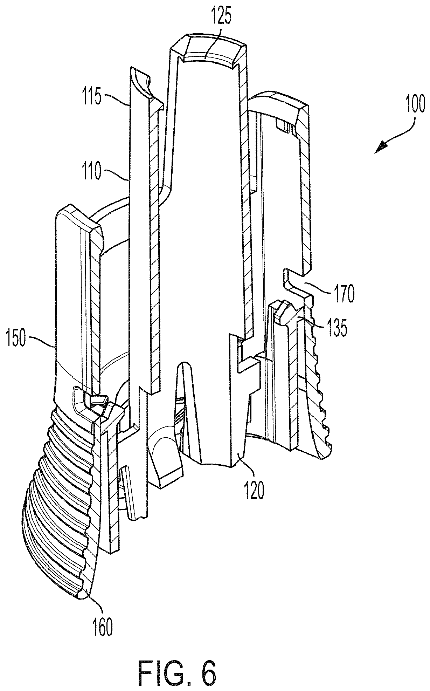

As described previously, proximal end (115) of retainer (110) can include at least one grasping feature, such as a projection, to allow secure engagement with a needle shield. Turning to **FIGS. 3** **and** **6**, shown is a cap assembly according to a non-limiting embodiment or aspect, where the retainer (110) includes at least one grasping feature (125) at a proximal end (115) thereof. The at least one grasping feature can be at least one protrusion (125), and can be configured such that it is capable of gripping a needle shield by a flange thereof.

In non-limiting embodiments or aspects, the retainer (110) and cap remover (150) of the cap assembly (100) are configured or arranged such that the needle shield and/or syringe of an autoinjector (not shown) can be shifted relative to the retainer by introducing a device, such as a tool, through the openings (122, 162) in the distal ends (120, 160) of the retainer (110) and cap remover (150), respectively. Placing a tool through the openings in the cap remover and retainer allows the needle shield and syringe to be displaced proximally, and thus allows for adjustment of the positioning of the projections (125) relative to the needle shield to ensure a secure grip between the retainer (110) and the needle shield.

With continued reference to **FIGS. 3** **and** **6**, as described above in non-limiting embodiments or aspects the cap remover (150) includes at least one opening (170) in a sidewall thereof. The at least one opening (170) is configured to allow at least one radially-extending protrusion (135) on retainer (110) to extend therethrough. In the non-limiting embodiment or aspect shown in **FIGS. 3** **and** **6**, at least one radially-extending protrusion (135) extends from an arm that extends proximally from the distal end (120) of the retainer (110). In other non-limiting embodiments or aspects (not shown), the at least one radially-extending protrusion (135) is positioned on a main body of the retainer (110).

With reference to **FIGS. 2A** **and** **2B****,** a lag arrangement is provided between the cap remover (150) and the retainer (110) during removal of the cap assembly (100). Retainer (110) is received within cap remover (150) such that at least one protrusion (112) is arranged proximally of at least one shoulder (152) of cap remover (150). In non-limiting embodiments or aspects, the at least one protrusion (112) prevents the retainer (110) from being displaced distally out of the cap remover (150), by virtue of engagement with a proximal surface of the at least one shoulder (152). In non-limiting embodiments or aspects, the retainer (110) further includes at least one flange (114), arranged distally of the at least one shoulder (152) of the cap remover (150). In this way, the retainer (110) is prevented from being displaced proximally out of the cap remover (150) by virtue of engagement of a proximal surface of the at least one flange (114) with a distal surface of the at least one shoulder (152). The longitudinal space between the at least one protrusion (112) and the at least one flange (114) on the retainer (110) results in a predetermined amount of "play" between these components. Accordingly, in non-limiting embodiments or aspects, when the cap remover (150) is pulled distally to remove the cap assembly (100) from a drug delivery device to which it is attached, cap remover (150) is pulled a certain distance before the at least one shoulder (152) engages the at least one protrusion (112) of the retainer (110). This results in a lag between the initiation of movement of the cap remover (150) and initiation of movement of the retainer (110). When cap remover (150) moves sufficiently such that the at least one shoulder (152) engages with the at least one protrusion (112), the retainer (110) begins to be pulled away from the drug delivery device, and, because of the positioning of the at least one projection (125) relative to the needle shield, the needle shield begins to be pulled away from the syringe and needle.

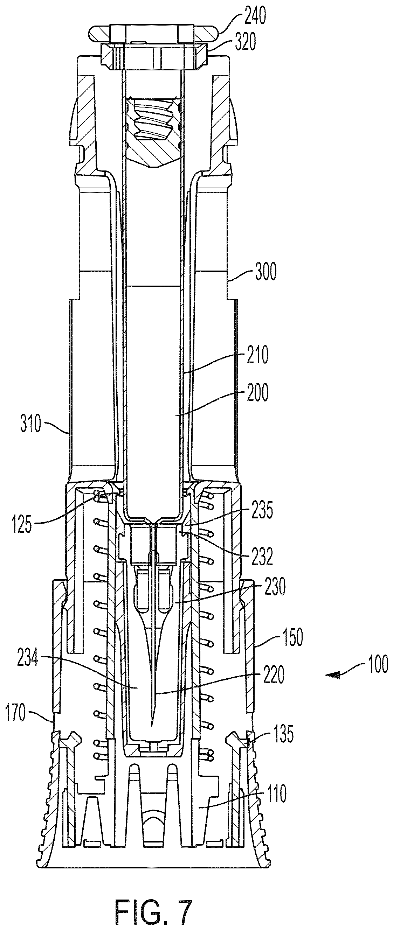

Also provided herein is an autoinjector including a housing, a syringe having a barrel, a needle, and a needle shield, and a cap assembly. Turning to **FIGS. 7-9**, shown is an autoinjector having a housing, a syringe (200) having a barrel (210) and a needle (220) attached thereto, a removable needle shield (230) configured to at least partially surround the needle (220), and a cap assembly (100). The autoinjector housing (a lower housing (310) is shown, though it should be understood that autoinjector can include an upper housing, as described below) includes a proximal end and a distal end, where the distal end is open to permit the needle (220) to pass therethrough to allow a substance to be delivered to a user.

Removable needle shield (230) can include proximal (232) and distal (234) ends. In non-limiting embodiments or aspects, removable needle shield (230) is a two-piece needle shield, including an elastomeric inner portion and a rigid outer portion. In non-limiting embodiments or aspects, the rigid outer portion at least partially surrounds the elastomeric inner portion of the needle shield (230). In non-limiting embodiments or aspects, needle shield (230) includes at least one flange or shoulder (235). In non-limiting embodiments or aspects, the at least one flange or shoulder (235) is located on the elastomeric inner portion, for engaging the rigid outer portion. In non-limiting embodiments or aspects, needle shield (230) includes at least one complementary grasping feature (233) on the proximal end (232) thereof. In non-limiting embodiments or aspects, the at least one complementary grasping feature (233) is configured to be complementary to, and engageable with, the at least one grasping feature (125) of the retainer. In non-limiting embodiments or aspects, the at least one complementary grasping feature (233) is the proximal edge of the needle shield (230).

In non-limiting embodiments or aspects, the elastomeric portion of the needle shield (230) is formed of one or more natural or synthetic rubbers and/or of thermoplastic elastomer, or combinations thereof. In non-limiting embodiments or aspects, the rigid outer portion of the needle shield (230) is formed of a rigid plastic. In non-limiting embodiments or aspects, the rigid outer portion is formed of polypropylene.

Cap assembly (100) includes, as described previously, cap remover (150) and retainer (110). In non-limiting embodiments or aspects, the cap remover (150) and/or retainer (110) include one or more of the features described above. In non-limiting embodiments or aspects, cap remover (150) at least partially surrounds the open distal end of the housing. In non-limiting embodiments or aspects, cap remover (150) is not rotatable relative to the housing. Autoinjector housing and/or cap remover (150) can include suitable features to prevent rotation of the cap remover (150), and, accordingly, retainer (110) and needle shield (230), relative to the housing and the needle (220), during needle shield (230) removal. In non-limiting embodiments or aspects, suitable anti-rotation features include one or more projections, guide channels, and/or shoulders on cap remover (150) and/or housing.

Also provided herein is a method of assembling an autoinjector including a cap assembly, including the steps of providing a lower housing assembly of an autoinjector, inserting a syringe into the lower assembly, and placing an upper assembly on the lower assembly. In a first step, a lower housing assembly (300), including cap assembly (100) engaged therewith, is provided. The lower housing assembly (300) includes lower housing (310). In non-limiting embodiments or aspects, lower assembly (300) includes a ring (320) at a proximal end thereof. The lower housing assembly (300) includes a proximal end and a distal end, the distal end being open to allow a needle of a syringe to pass therethrough, and the proximal end of the lower housing (310) being open to allow a syringe to be inserted into the housing. Housing may be formed of any suitable material, including, for example and without limitation, a rigid plastic. Lower assembly (300) also includes a cap assembly (100). Cap assembly (100) includes, as described previously, cap remover (150) and retainer (110), each having distal ends (160, 120) having an opening (162, 122) therethrough. In non-limiting embodiments or aspects, the cap remover (150) and/or retainer (110) include one or more of the features described above. In an initial configuration (e.g., when cap assembly (100) is positioned on lower housing (310)), the distal end (120) of the retainer (110) is spaced from the distal end (160) of the cap remover (150).

With reference to **FIG. 7**, in non-limiting embodiments or aspects, in a second step of the method, a syringe (200) is inserted into the lower housing (310). The syringe (200) can include a barrel (210) to hold a medicament or other substance therein, a needle (220) arranged at a distal end of the barrel (210), and a removable needle shield (230). Syringe (200), barrel (210), needle (220), and needle shield (230) can include one or more of the features described above. Syringe barrel (210) can be of any useful size, and can be formed of any useful material, such as glass or plastic. In non-limiting embodiments or aspects, syringe (200) is a unitary syringe, and the needle (220) is formed with the syringe barrel (210). In non-limiting embodiments or aspects, syringe barrel (210) is not formed with needle (220) therein and, instead, a needle hub (not shown) holds needle (220). Syringe needle (220) can be formed of any useful material, and can be of any suitable gauge. Needle shield (230) can be a one-piece needle shield formed of an elastomeric material, or a two-piece needle shield having an inner portion formed of an elastomeric material and an outer portion formed of a rigid material. The needle shield (230) at least partially surrounds the needle (220). In non-limiting embodiments or aspects, syringe (200) includes a flange (240) at a proximal end thereof. At the conclusion of the second step, the distal end (120) of the retainer (110) remains longitudinally spaced from the distal end (160) of the cap remover (150).

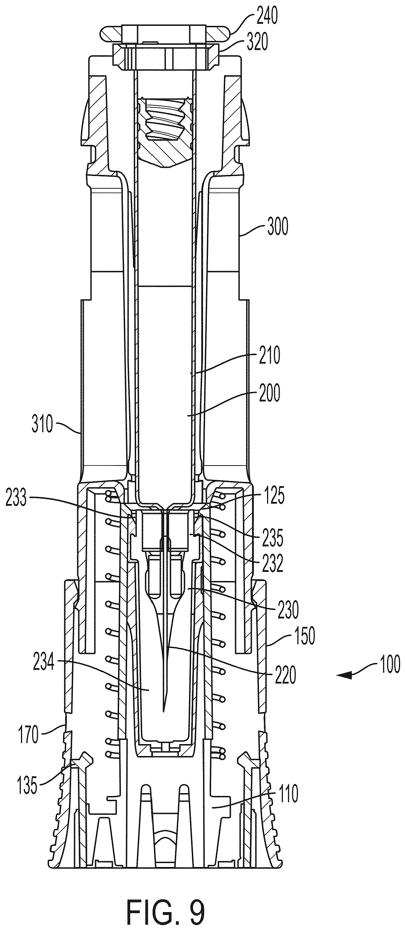

In non-limiting embodiments or aspects, the step of inserting the syringe (200) includes moving the syringe (200) towards the distal end of the housing (310), preferably such that the at least one grasping feature (125) is spaced longitudinally from the proximal end of the needle shield. In other words, the at least one grasping feature (125) is more proximal than in its final position (shown in **FIG. 9**). In this position, the distal end (234) of the syringe flange abuts the ring (320). In this position, the proximal end (115) of the retainer (110) contacts the syringe barrel (210).

As described above, at the conclusion of this step, the distal end (120) of the retainer (110) is maintained proximally with respect to the distal end (160) of the cap remover (150), such that the distal end (120) of the retainer (110) is longitudinally spaced from the distal end (160) of the cap remover (150). In non-limiting embodiments or aspects, a tool (not shown) is inserted within the opening (162) of the cap remover (150). This tool can be used to push the retainer (110) proximally, such that the distal end (120) of the retainer (110) is longitudinally spaced from the distal end (160) of the cap remover (150).

In non-limiting embodiments or aspects, the proximal end (115) of the retainer (110) includes at least one grasping feature, such as projections (125), and the projections (125) contact the syringe barrel (210) proximally of the distal end of the syringe barrel (210). In this way, the retainer (110) is in an "overstroked" position, where the at least one grasping feature (125) is located too far proximally of the needle shield (230) (**FIG. 7**). In non-limiting embodiments or aspects, the needle shield (230) includes at least one flange or shoulder (235), and the proximal end (115) of the retainer (110) includes at least one grasping feature (125), and the at least one grasping feature (125) contacts the syringe barrel (210) proximally of the at least one flange or shoulder (235).

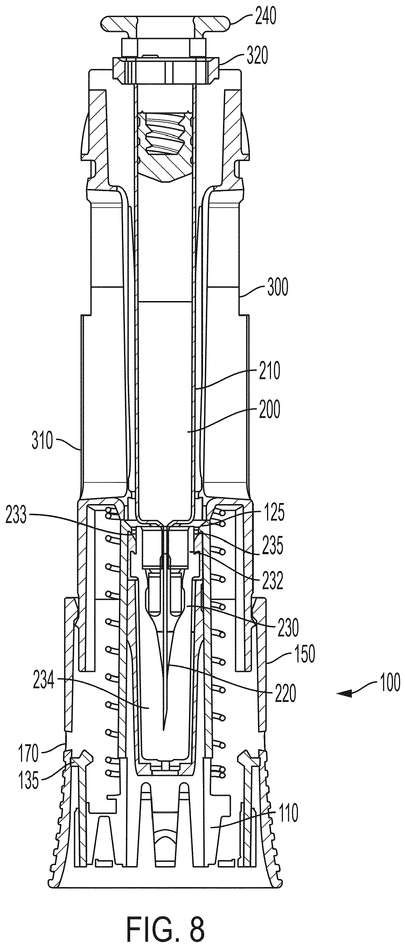

With reference to **FIG. 8****,** in non-limiting embodiments or aspects, a third step of the method of assembling the syringe includes, after inserting the syringe (200) into the lower housing (310), moving the syringe (200) proximally. In non-limiting embodiments or aspects, moving the syringe (200) proximally within the lower housing (310) is achieved by inserting a device through the at least one opening (162, 122) in the distal ends (160, 120) of the cap remover (150) and retainer (110), and applying pressure to urge the syringe (200) proximally. In non-limiting embodiments or aspects, and as shown in **FIG. 8**, when the syringe (200) is urged proximally, the at least one grasping feature (125) of the retainer (110) engages the needle shield at a proximal-most end thereof. In non-limiting embodiments or aspects, the at least one grasping feature (125) engages at least one corresponding, complementary grasping feature (233) on the needle shield (230). In non-limiting embodiments or aspects, after the syringe (200) is urged proximally, the syringe flange (240) is longitudinally spaced from the ring (320) of the lower assembly (300). At the end of this step the at least one grasping feature (125) engages the at least one complementary grasping feature (233) of the needle shield (230), the distal end (120) of the retainer (110) is longitudinally spaced from the distal end (160) of the cap remover (150), and the syringe flange (240) is longitudinally spaced from the ring (320).

In non-limiting embodiments or aspects, the method includes a fourth step of placing an upper assembly (not shown), including an upper housing, on the lower assembly. As with lower assembly and lower housing, upper assembly and upper housing can be formed of any suitable material, including, for example and without limitation, a rigid plastic. In non-limiting embodiments or aspects, the step of placing the upper assembly on the lower assembly causes the syringe to move distally, such that the syringe flange (240) abuts the ring (320).

With reference to **FIG. 9**, after assembly, distal end (120) of retainer (110) falls or is otherwise displaced towards the distal end (160) of the cap remover (150), such that the distal end (120) of the retainer (110) abuts the distal end (160) of the cap remover (150).

Once a final user wants to remove the needle shield, he/she pulls the cap remover. As described above, the gap between the at least one protrusion (112) of the retainer (110) and the at least one shoulder (152) of the cap remover (150) causes a lag between the initiation of longitudinal movement of the cap remover (150) away from the lower housing assembly (300) and the initiation of longitudinal movement of the retainer (110) away from the lower housing assembly (300).

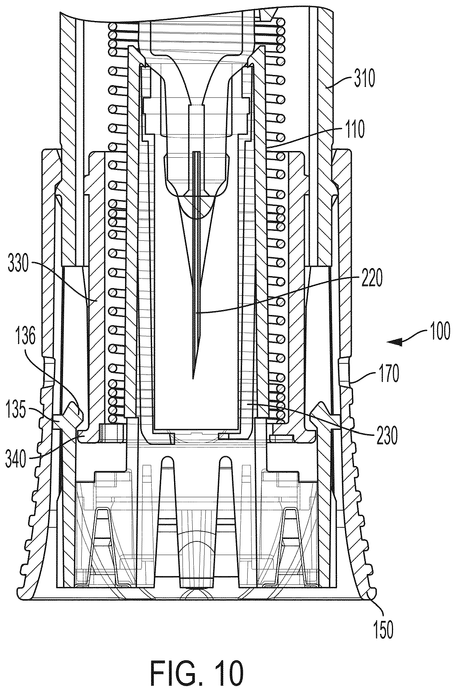

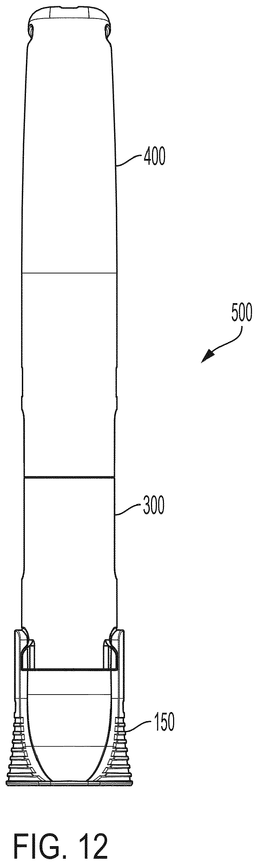

Turning to **FIG. 10****,** in non-limiting embodiments or aspects, the cap assembly (100) functions to prevent actuation of a drug delivery device, such as an autoinjector, to which the cap assembly (100) is attached. With reference to **FIG. 12**, shown is one non-limiting embodiment or aspect of an autoinjector with which the present assembly can be utilized. Autoinjector (500) includes lower housing assembly or subassembly (300) as described previously, an upper housing subassembly (400), cap remover (150), and retainer (110). The lower subassembly includes housing (310) and a needle cover (330). In the non-limiting embodiments or aspects shown in **FIGS. 10-12****,** needle cover (330) is configured to shield the needle (220) of the syringe (200) from a user when, for example, the removable needle shield (230) has been removed. Needle cover (330) is moveable from an extended position (pre-use), to a retracted position (use), where the needle cover (330) translates proximally into the lower housing assembly (300), to a second extended position (post-use). In both extended positons (pre- and post-use), the needle cover (330) at least partially shields the needle (220). In the use position, the needle cover (330) does not shield the needle, which can be inserted into a user's skin.

In the non-limiting embodiments or aspects shown in **FIGS. 10-12****,** in order to use the autoinjector (500), a user removes the cap assembly (100) as described above (and below) and applies the distal end of the autoinjector (500) to the skin at the site of the intended injection. Needle cover (330) is positioned at the distal end of the autoinjector, and thus contacts the skin of the user. Application of force to the autoinjector (500) towards the skin causes movement of the needle cover (330) proximally into the housing (310) of the lower housing subassembly. In non-limiting embodiments or aspects, movement of the needle cover (330) proximally allows for delivery of the medicament, for example through interaction of the needle cover (330) with a container holder (not shown). Specifically, in non-limiting embodiments or aspects, proximal movement of the needle cover (330) causes the needle cover (330) to, at its proximal end (335), contact a container holder provided in the upper housing subassembly (400), which subsequently causes the container holder to be displaced proximally. The container holder can have at least one window (not shown) that allows biased blocking elements (not shown), which hold the plunger (not shown) of the autoinjector (500) in a pre-use position against the biasing force of an injection spring (not shown), to move radially to an unbiased position. The radial movement of the blocking elements releases the plunger, which is displaced out of the container holder by the injection spring, which moves a piston (not shown) in the syringe barrel (210) to cause injection of medicament through the needle (220).

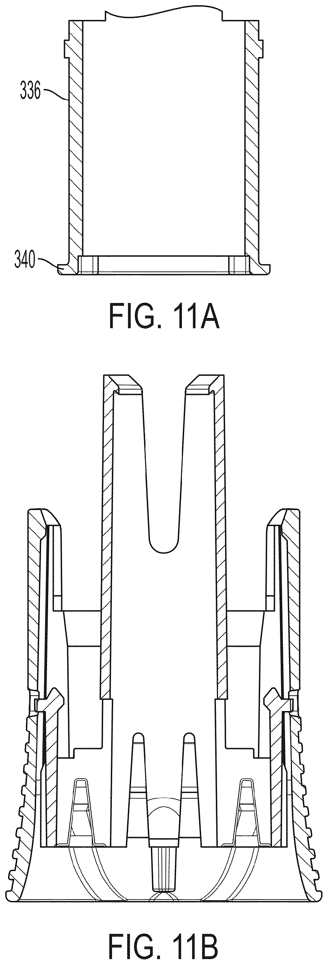

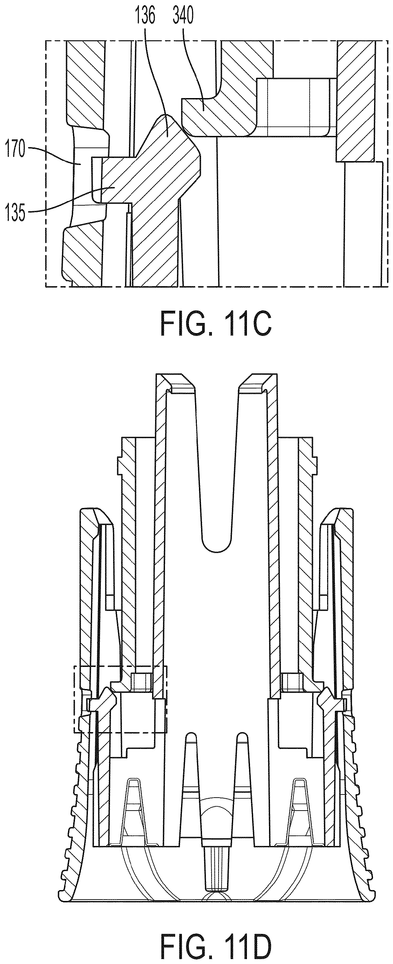

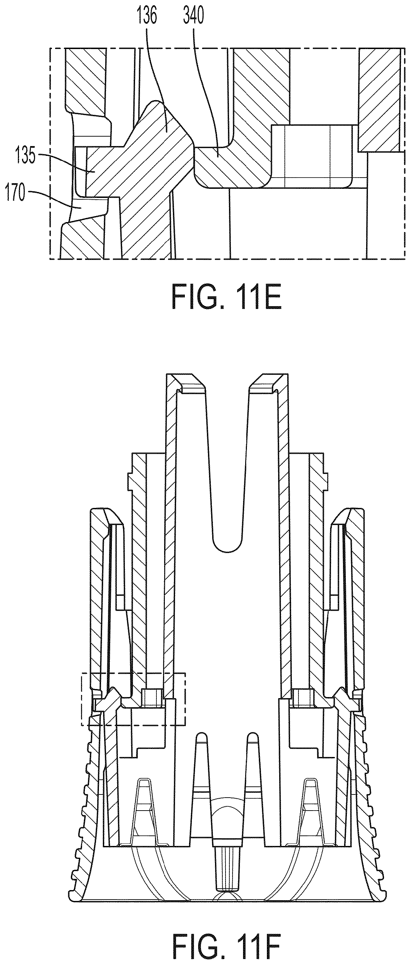

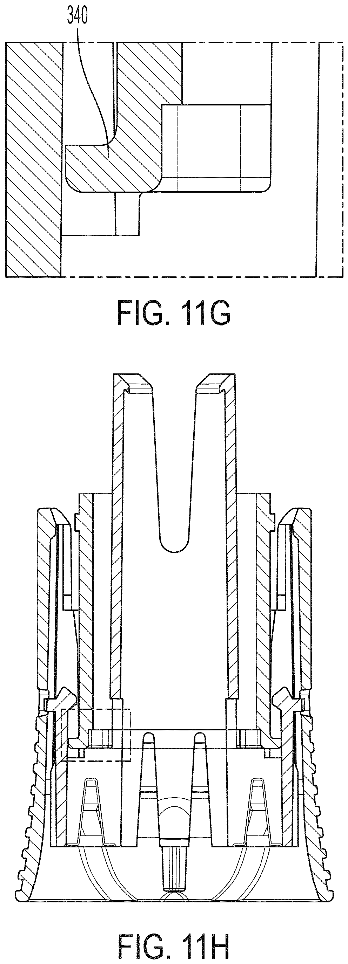

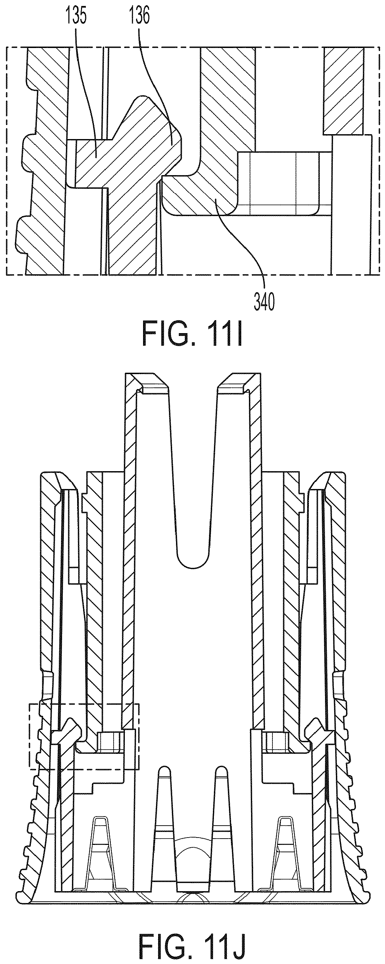

As can be appreciated from the above-described embodiment or aspect, proximal movement of the needle cover (330) can cause unintended actuation of the autoinjector (500). Accordingly, as shown in **FIGS. 10** **and** **11****,** in non-limiting embodiments or aspects provided is a subassembly for a drug delivery device, such as an autoinjector. The subassembly includes a housing (310), needle cover (330), and cap assembly (100) including retainer (110) and cap remover (150). In non-limiting embodiments or aspects, the retainer (110) prevents unintended proximal movement of the needle cover (330), and thus unintended actuation of a drug delivery device of which the subassembly is a component. In non-limiting embodiments or aspects, the retainer (110) includes at least one protrusion, such as inward-facing protrusion (136). In non-limiting embodiments or aspects, the at least one inward-facing protrusion (136) is arranged on the same proximally-extending arm on which at least one radially-extending protrusion (135) is positioned.

With continuing reference to **FIGS. 10** **and** **11****,** in non-limiting embodiments or aspects, the needle cover (330) includes at its distal end a lip or flange (340), protruding outwardly. In non-limiting embodiments or aspects, inward-facing protrusion (136) on retainer (110) is located proximally of the lip or flange (340) of the needle cover (330), preventing the needle cover (330) from moving proximally a distance sufficient to allow actuation of the autoinjector (500). In non-limiting embodiments or aspects, inward-facing protrusion (136) engages or abuts the lip or flange (340) of the needle cover (330), substantially or completely preventing proximal movement of the needle cover (330).

**FIG. 10** shows a non-limiting embodiment or aspect in which lip or flange (340) is arranged on an outer surface of needle cover (330), and protrusion (136) is an inward-facing protrusion configured to engage the lip or flange (340). In non-limiting embodiments or aspects (not shown), the protrusion is an outward-facing protrusion and is arranged on an arm that extends proximally from the distal end of the retainer (110) through an interior space defined by the needle cover (330), and is configured to engage a lip or flange arranged on an inner surface of the needle cover.

Turning to **FIG. 11****,** shown is the aforementioned assembly process, with focus on the interaction between the at least one inward-facing protrusion (136) and the lip or flange (340) of the needle cover (330), and the at least one radially-extending protrusion (135) and the at least one opening (170).

**FIGS. 11A and 11B** show needle cover (330) (graphically isolated from remainder of lower housing assembly (300)), having lip or flange (340), and cap assembly including cap remover and retainer, prior to placement of the needle cover into the cap assembly.

**FIGS. 11C and 11D** show an initial step of assembly, when the cap assembly is first placed on the needle cover. The at least one radially-extending protrusion (135) of the retainer is capable of deflecting outwardly due to its longitudinal positioning with regard to the at least one opening (170). Lip or flange (340) of the needle cover is engaged with a proximal-facing surface of the at least one inward-facing protrusion (136).

**FIGS. 11E and 11F** show a further step in the assembly process. Lip or flange (340) has begun to move distally past the at least one inward-facing protrusion (136). In non-limiting embodiments or aspects, where the at least one inward-facing protrusion (136) is positioned on at least one arm of the retainer, the arm(s) can flex outward as the lip or flange (340) passes by the at least one protrusion (136), allowing the needle cover to move distally past the protrusion(s) (136).

With continuing reference to **FIG. 11****,** **FIGS. 11G and 11H** show a further step in the assembly process, where the lip or flange (340) has passed distally by the at least one inward-facing protrusion (136). In non-limiting embodiments or aspects in which the at least one inward-facing protrusion (136) can flex outward to allow lip or flange (340) to pass distally, the arm(s) have returned to an unbiased state.

As described previously, after the lower housing (310) is assembled on the needle cover and cap assembly, the retainer moves distally (**FIGS. 11I and 11J**). By virtue of the arrangement of the assembly of the lower housing (310), the protrusions (135) are prevented from moving so that they are aligned with the openings (170). Consequently, the arms, including protrusions (135) are prevented from flexing outwardly as long as the cap assembly (100) is assembled on the lower housing assembly (300). The inward facing protrusions (136) are consequently maintained in engagement with the flange (340) of the needle cover (330). This engagement prevents proximal translation of the needle cover (330), which, as described above, prevents actuation of the autoinjector. In this way, unintended actuation of the device can be avoided.

To be able to use the autoinjector, the user has to first remove the cap assembly (100). To do so, he/she pulls the cap remover (150) away from the lower housing (310). Because of the axial "play" between the retainer (110) and the cap remover (150), the cap remover (150) will move with respect to the retainer (110), such that the at least one protrusion (135) is again aligned with the at least one opening (170), thereby allowing deflection of the arm of the retainer (110) outward and disengagement between the inward facing protrusion (136) and the flange (340) of the needle cover (330). This allows disengagement between the retainer (110) and the needle cover (330). The needle cover (330) is then free to move proximally to trigger an injection.

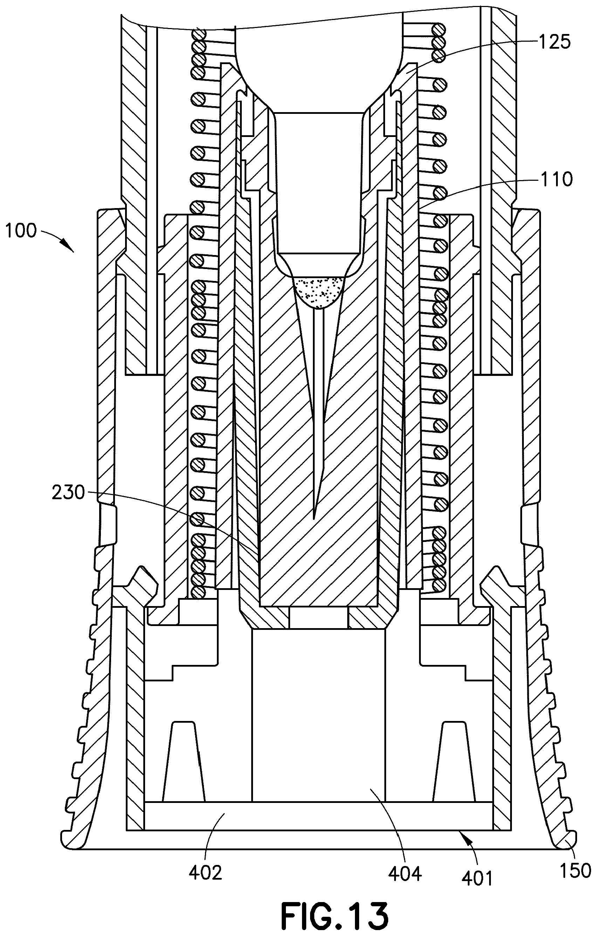

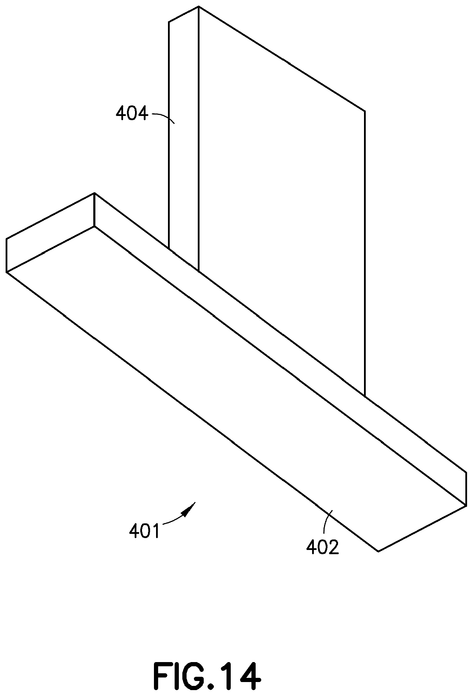

With reference to **FIGS. 13** and **14****,** according to one non-limiting embodiment or aspect of the present disclosure, another embodiment of the cap assembly (100) is shown and described in detail. The distal end (160) of cap remover (150) may include a push back element (401) positioned in the at least one opening (162). In one example, the push back element (401) may be removably connected or positioned in the opening (162). In another example, the push back element (401) may be integrally formed with the cap remover (150) in the cap assembly (100). The push back element (401) may be made of a medical-grade plastic or a material similar to the cap assembly (100).

In one non-limiting embodiment or aspect, the push back element (401) may include a tab (402) and an extension member (404) that extends from the tab (402). The tab (402) and the extension member (404) may be integral formed with one another. The extension member (404) may be received in the opening (122) defined in the distal end (120) of retainer (110) of the cap assembly (100). The cross-sectional shape of the extension member (404) may correspond to the cross-sectional shape of the opening (122) in a keyhole configuration. The extension member (404) may be removably held in the opening (122) or formed integral with the retainer (110). The extension member (404) may be held in the retainer (110) using a friction fit, a snap fit, a welded connection, an adhesive connection, or any other connection that allows the push pack element (401) to be held in the retainer (110).

With continued reference to **FIG. 13****,** a proximal surface of the tab (402) is configured to rest against the distal end of the retainer (110) once the extension member (404) has been inserted into the retainer (110). When inserted into the retainer (110), the tab (402) may be pushed by the user to push back or move the retainer (110) in a proximal direction. Based on this movement in the proximal direction, the grasping features (125) of the retainer (110) are moved in a proximal direction to ensure connection with the proximal end of the removable needle shield (230). The proximal surface of the tab (402) contacts and forces the retainer (110) in a proximal direction, thereby ensuring the grasping features (125) move far enough in the proximal direction to ensure the grasping features (125) move past the proximal end of the removable needle shield (230) to ensure the grasping features (125) engage the proximal end of the removable needle shield (230) when the cap assembly (100) is pulled in a distal direction.

By providing the push back element (401) with the cap assembly (100), it becomes more intuitive to the user as to the specific location that should be pushed on the cap assembly (100) to move the retainer (100) in the proximal direction. Without the push back element (401) held in the retainer (110), the empty opening (162) of the cap assembly (100) often confuses users as to where pressure should be applied to the cap assembly (100). The push back element (401) also provides a better aesthetic finish to the cap assembly (100) rather than the large opening (162) that remains otherwise. In one example of the present disclosure, the tab (402) of the push back element (401) may have a diameter that substantially corresponds to the diameter of the opening (162) such that the opening (162) is covered by the tab (402). By covering the opening (162) of the cap remover (150) with the tab (402), any confusion from the user as to whether the drug delivery device (500) is ready for use with the cap remover (150) still affixed to the housing may be reduced since the tab (402) may indicate whether the retainer (110) has been pushed in the proximal direction due to the relative position of the tab (402) in the opening (162).

Although the invention has been described in detail for the purpose of illustration based on what is currently considered to be the most practical and preferred embodiments, it is to be understood that such detail is solely for that purpose and that the invention is not limited to the disclosed embodiments, but on the contrary, is intended to cover modifications and equivalent arrangements that are within the spirit and scope of the appended claims. For example, it is to be understood that the present invention contemplates that, to the extent possible, one or more features of any embodiment can be combined with one or more features of any other embodiment.

## Claims

1. A subassembly for a drug delivery device comprising:
a housing (310);
a displaceable needle cover (330) comprising a distal end comprising a flange (340), the needle cover (330) received within the housing (310) and configured to have a pre-use position in which the needle cover (330) extends from the housing (310) and a use position in which the needle cover (330) is at least partially displaced into the housing (310), wherein displacement of the needle cover (330) into the housing (310) actuates the drug delivery device through interaction with a drive mechanism; and
a cap assembly (100) comprising:
a cap remover (150) configured to be grasped by a user;
a retainer (110) received within the cap remover (150) and configured to engage the flange (340) of the needle cover (330) of the drug delivery device; and
a push back element (401) operatively received within the retainer (110),
wherein the push back element (401) is configured to push the retainer (110) in a proximal direction.

2. The subassembly of claim 1, wherein the push back element (401) comprises a tab (402) and an extension member (404) that extends from the tab (402).

3. The subassembly of claim 1 or claim 2, wherein the push back element (401) is removably held in the retainer (110).

4. The subassembly of any of claims 1-3, wherein the push back element (401) comprises the tab (402) that covers an opening defined in the cap remover (150).

5. The subassembly of any of claims 1-4, wherein the push back element (401) comprises the extension member (404) configured to be received in the retainer (110), wherein at least a portion of the retainer (110) defines an opening with a cross-sectional shape that corresponds to a cross-sectional shape of the extension member (404).

6. The subassembly of any of claims 1-5, wherein the push back element (401) is made of a medical-grade plastic.

7. The subassembly of any of claims 1-6, wherein a proximal surface of the push back element (401) is configured to push the retainer (110) in the proximal direction.

8. The subassembly of any of claims 1-7, wherein the push back element (401) comprises the tab (402) that is shaped to correspond to a shape of an opening defined in the cap remover (150).

9. A drug delivery device (500) comprising:
a housing (310);
a syringe (200) comprising a needle (220) and received at least partially within the housing (310);
a needle cover (330) comprising a flange (340) at a distal end thereof and having a pre-use position in which the needle (220) is positioned within the needle cover (330) and a use position in which the needle (220) is positioned at least partially outside of the needle cover (330), wherein in the use position the needle cover (330) is configured to actuate the drug delivery device to deliver a medicament; and
a cap assembly (100) comprising a cap remover (150), a retainer (110) received within the cap remover (150) and a push back element (401) operatively received within the retainer (110), wherein the push back element (401) is configured to push the retainer (110) in a proximal direction.

10. The drug delivery device of claim 9, wherein the push back element (401) comprises a tab (402) and an extension member (404) that extends from the tab (402).

11. The drug delivery device of claim 9 or claim 10, wherein the push back element (401) is removably held in the retainer (110).

12. The drug delivery device of any of claims 9-11, wherein the push back element (401) comprises the tab (402) that covers an opening defined in the cap remover (150).

13. The drug delivery device of any of claims 9-12, wherein the push back element (401) comprises the extension member (404) configured to be received in the retainer (110), wherein at least a portion of the retainer (110) defines an opening with a cross-sectional shape that corresponds to a cross-sectional shape of the extension member (404).

14. The drug delivery device of any of claims 9-13, wherein the push back element (401) is made of a medical-grade plastic.

15. A cap assembly (100) for a drug delivery device (500), the cap assembly (100) comprising:
a cap remover (150) configured to be grasped by a user;
a retainer (110) received within the cap remover (150) and configured to engage the flange (340) of the needle cover (330) of the drug delivery device; and
a push back element (401) operatively received within the retainer (110),
wherein the push back element (401) is configured to push the retainer (110) in a proximal direction.

16. The cap assembly of claim 15, wherein the push back element (401) comprises a tab (402) and an extension member (404) that extends from the tab (402).

17. The cap assembly of claim 15 or claim 16, wherein the push back element (401) is removably held in the retainer (110).

18. The cap assembly of any of claims 15-17, wherein the push back element (401) comprises the tab (402) that covers an opening defined in the cap remover (150).

19. The cap assembly of any of claims 15-18, wherein the push back element (401) comprises the extension member (404) configured to be received in the retainer (110), wherein at least a portion of the retainer (110) defines an opening with a cross-sectional shape that corresponds to a cross-sectional shape of the extension member (404).

20. The cap assembly of any of claims 15-19, wherein the push back element (401) is made of a medical-grade plastic.
